# EUROPÄISCHE PATENTANMELDUNG

(11) **EP 1 897 589 A2**
(43) Veröffentlichungstag der Anmeldung: **12.03.2008**
(21) Anmeldenummer: 07015670.8
(22) Anmeldetag: 09.08.2007
(51) Int. Cl.: A61N 1/375

(54) **Elektrische Durchführung**

(30) Priorität: 07.09.2006 DE 102006041940
(71) Anmelder: BIOTRONIK CRM Patent AG, 6341 Baar (CH)
(72) Erfinder: Teske, Josef, 96103 Hallstadt (DE); Eck, Stefan, 91315 Höchstadt (DE); Frauenstein, Boris, 91074 Herzogenaurach (DE); Haas, Erich, 91604 Flachslanden (DE)
(74) Vertreter: Lindner-Vogt, Karin L.

(57) **Zusammenfassung**

Die Erfindung betrifft eine elektrische Durchführung zum Einsetzen in eine Öffnung eines implantierbaren elektrischen Therapiegerätes mit einem elektrisch isolierenden Isolationskörper, durch den wenigstens ein elektrisch leitender Anschlusspin hindurchtritt, der mittels eines Lots hermetisch dicht mit dem Isolationskörper verbunden ist, wobei das Lotmaterial Glas oder Glaskeramik ist.

## Beschreibung

Die Erfindung betrifft eine elektrische Durchführung zum Einsetzen in eine Öffnung eines implantierbaren elektrischen Therapiegerätes. Solche elektrischen Therapiegeräte sind beispielsweise implantierbare Herzschrittmacher, implantierbare Kardioverter/Defibrillatoren oder Kochlea-Implantate.

Die elektrische Durchführung besitzt einen elektrisch isolierenden Isolationskörper, durch den wenigstens ein elektrisch leitender Anschlusspin hindurchtritt, der mittels eines Lots hermetisch dicht mit dem Isolationskörper verbunden ist.

Derartige elektrische Durchführungen dienen dazu, eine elektrische Verbindung zwischen einem hermetisch abgeschlossenen Inneren eines Therapiegerätes und dem Äußeren des Therapiegerätes herzustellen. Bei bekannten E-lektrotherapiegeräten, wie beispielsweise Herzschrittmachern oder Defibrillatoren, ist üblicherweise ein hermetisch dichtes Metallgehäuse vorgesehen, welches auf einer Seite einen Anschlusskörper, auch Header genannt, aufweist, der Anschlussbuchsen für das Anschließen von Elektrodenleitungen trägt. Die Anschlussbuchsen weisen elektrische Kontakte auf, die dazu dienen, Elektrodenleitungen elektrisch mit der Steuerelektronik im Inneren des Gehäuses des Herzschrittmachers zu verbinden. Dort, wo die elektrische Verbindung in das Gehäuse des Herzschrittmachers eintritt, ist üblicherweise eine Durchführung vorgesehen, die hermetisch dichtend in eine entsprechende Gehäuseöffnung eingesetzt ist.

Häufig sind derartige elektrische Durchführungen als Filterdurchführungen ausgebildet. In diesem Falle tragen die Vorrichtungen ein elektrisches Filter, das dazu dient, externe hochfrequente elektrische Störungen kurzzuschließen, so dass entsprechende Signale der Steuerelektronik im Inneren des Gehäuses wenn überhaupt nur stark gedämpft zugeführt werden und die Steuerelektronik erst bei wesentlich größeren Signalstärken der elektrischen Störung gestört wird, als dies ohne elektrisches Filter der Fall wäre. Ein derartiges Filter wird üblicherweise von einem Filterkörper gebildet, der nach Art eines Kondensators zwischen eine Gerätemasse und eine jeweilige, durch die Durchführung hindurchtretende, elektrische Leitung geschaltet ist.

Eine solche, durch die Durchführung hindurchtretende elektrische Leitung, wird üblicherweise von einem elektrisch leitenden Anschlusspin gebildet, der durch eine Durchgangsöffnung in einem elektrisch isolierenden Isolationskörper hindurchtritt, und der elektrisch leitende Anschlusspin steht auf beiden Seiten über die jeweilige Stirnfläche des Isolationskörpers über, so dass auf beiden Seiten des Isolationskörpers - und damit auf beiden Seiten der elektrischen Durchführung - jeweils weiterführende elektrische Leitungen an den Anschlusspin - beispielsweise durch Löten oder Schweißen - angeschlossen werden können. Ein möglicher Spalt zwischen einer Durchgangsöffnung im Isolationskörper, durch den ein jeweiliger Anschlusspin hindurchtritt und dem Anschlusspin selbst, wird üblicherweise mit einem Lot, in der Regel Goldlot, hermetisch dicht verschlossen.

Eine Vielzahl derartiger elektrischer Durchführungen sind aus dem Stand der Technik bekannt. Beispiele sind in US 6,934,582, US 6,822,845, US 6,765,780, US 6,643,903, US 6,567,259, US 6,768,629, US 6,765,779, US 6,566,978 und US 6,529,103 zu finden.

Trotz der Vielzahl bekannter Durchführungen besteht nach wie vor der Bedarf, diese hinsichtlich Herstellbarkeit und Eigenschaften zu verbessern.

Erfindungsgemäß wird dieses Ziel dadurch erreicht, dass als Material des Lots zwischen Isolationskörper und Anschlusspin Glas oder Glaskeramik vorgesehen ist. Insbesondere bei der Verwendung von keramischen Isolationskörpern ergibt sich gleichzeitig eine Reduktion der Herstellungskosten und eine Verbesserung der Zuverlässigkeit dadurch, dass ein Lot direkt sowohl mit dem Isolationskörper als auch mit dem Anschlusspin und ggf. dem Flansch verbunden werden kann, ohne dass es dazu aufwändiger Vorbereitungsarbeiten, beispielsweise bei der Keramikherstellung oder einer Beschichtung des Isolationskörpers bedarf.

Dies bringt den Vorteil mit sich, dass die Anzahl der Komponenten und Prozessschritte bei der Herstellung reduziert ist.

Ein weiterer wichtiger Vorteil ist, dass das Lotmaterial Glas oder Glaskeramik elektrisch isolierend ist und dadurch gleichzeitig mit dem Flansch und dem Pin verbunden sein darf. Bei einem leitfähigen Lot wie beispielsweise Gold benötigen Pin und Flansch dagegen mindestens zwei getrennte Lotreservoire, da es ansonsten zwischen Pin und Flansch zu einem elektrischen Kurzschluss kommen würde. Somit ermöglicht ein elektrisch isolierendes Lot wie Glas oder Glaskeramik einfachere und kompaktere Konstruktionen von elektrischen Durchführungen.

Auch lässt sich auf diese Weise ohne weiteres eine biokompatible Oberfläche des Isolationskörpers auf seiner Außenseite (bezogen auf den eingebauten Zustand) erzielen.

Der letztgenannte Vorteil ist insbesondere dann gegeben, wenn der Isolationskörper aus einem keramischen Material besteht, welches vorzugsweise Al₂O₃ enthält.

Der Grad der Biokompatibilität wird noch dadurch gesteigert, wenn das Lotmaterial Glas oder Glaskeramik biokompatibel ausgeführt ist und/oder der keramische Isolationskörper und/oder der Flansch so geformt sind, dass ein potentieller Zutritt von Körperflüssigkeit zum Lot über eine oder mehrere eng geführte Kanten zusätzlich erschwert wird.

Der Lotverlauf des Glases oder der Glaskeramik wird kontrollierbarer, wenn neben dem körperzugewandten Isolationskörper ein weiterer Isolationskörper auf der anderen Seite des Glas- oder Glaskeramiklotes mit verlötet wird, so dass das Glas- oder Glaskeramik-Lot in der Flanschbohrung von beiden Seiten von Isolationskörpern eingeschlossen ist und beide Isolationskörper zusammen mit dem Flansch und dem Pin verlötet.

Die Durchführung wird für Hochspannungsanwendungen wie bei Defibrillatoren besonders geeignet, wenn die Isolationskeramik so ausgeformt wird, dass lange Isolationsstrecken auf der Oberfläche und im Volumen entstehen. Geeignete Ausformungen sind z. B. Ausbuchtungen und Kanten. Solche Ausformungen werden vorzugsweise auf der körperzugewandten Seite der Durchführung ausgebildet.

Entsprechend ist es ein auch unabhängig von den übrigen hier beschriebenen Merkmalen einer Durchführung zu verwirklichender, selbstständiger Gedanke, den Isolationskörper so zu Formen, dass er lange Isolationsstrecken auf der Oberfläche und im Volumen bietet, also z.B. eine Oberfläche mit entsprechenden Vertiefungen oder Erhebungen aufweist, die der Verlängerung der Isolationsstrecke dienen.

Zudem bieten derartige Ausformungen stabile Verankerungsmöglichkeiten für den Header, so dass dessen Anbindung an das Gehäuse des Implantats sicherer wird.

Der Anschlusspin besteht vorzugsweise aus Metall, welches vorzugsweise Platin enthält und besonders bevorzugt eine Platin-Iridium-Legierung ist. Als weitere, besonders biokompatible und korrosionsbeständige Metalle für den Pin kommen Niob, Tantal und Titan und deren geeignete Legierungen in Betracht. Derartige Anschlusspins weisen die gewünschte Biokompatibilität auf, sind korrosionsbeständig und lassen sich zuverlässig verarbeiten.

In einer bevorzugten Ausführungsvariante sind der Anschlusspin oder die Anschlusspins jeweils in eine Durchgangsbohrung in dem Isolationskörper eingesetzt und mit diesem durch das von Glas bzw. Glaskeramik gebildete Lot mechanisch fest und hermetisch dicht verbunden. Dabei besitzt der Flansch entweder für jeden Pin eine separate Durchgangsbohrung oder mehrere Pins teilen sich eine gemeinsame Durchgangsbohrung.

In einer weiteren Ausführungsvariante besitzt jeder Pin seinen separaten Isolationskörper mit dem Vorteil, dass die Isolationskörper rotationssymmetrisch, z. B. zylindrisch ausgebildet sein können und einfach herstellbar sind.

Zur Verbesserung der Lotverbindung zwischen Anschlusspin und Isolationskörper kann eine entsprechende Durchgangsöffnung für den Anschlusspin wenigstens an einem Längsende eine Aufweitung aufweisen, so dass zwischen Anschlusspin und erweiterter Durchgangsöffnung ein Raum entsteht, der mit Glas- oder Glaskeramik-Lot gefüllt ist. Der beschriebene Raum kann vorzugsweise als Ringraum ausgebildet sein und wird im Folgenden der Einfachheit halber als Kavität bezeichnet; es sei ausdrücklich darauf hingewiesen, dass die Kavität auch jede andere Form annehmen kann. Beispielsweise können sich die Räume überschneiden und einen gemeinsamen Raum ausbilden, der mit Glas- oder Glaskeramik-Lot gefüllt ist.

Um für Therapiegeräte geeignet zu sein, deren elektrische Komponenten im Inneren des Gehäuses über mehrere elektrische Leitungen, beispielsweise mit einer oder mehreren Elektrodenleitungen zu verbinden sind, ist die Durchführung vorzugsweise mehrpolig ausgebildet und weist hierzu mehrere, vorzugsweise parallel zueinander verlaufende Anschlusspins und eine entsprechende Anzahl von Durchgangsbohrungen auf. Diese Durchgangsbohrungen besitzen vorzugsweise jeweils nur an einem Längsende einen Durchmesser, der deutlich größer ist, als der Außendurchmesser des Anschlusspins, so dass zwischen Anschlusspin und Bohrung eine Kavität entsteht. Diese Kavitäten sind vorzugsweise sämtlich auf derselben Stirnseite des Isolationskörpers angeordnet.

Das Anschließen von elektrischen Leitungen eines Headers wird erleichtert, wenn die Anschlusspins auf der Außenseite der Durchführung (bezogen auf den eingebauten Zustand) eine unterschiedliche Länge aufweisen. Das Anschließen der elektrischen Leitungen wird in machen Fällen weiter erleichtert, wenn die Pins an ihren Enden abgeflacht, gebogen, in Form von Nägelköpfen oder in sonstige geeignete Formen gebracht werden.

Um bei einem möglichst kleinen Isolationskörper einen möglichst großen Abstand der Anschlusspins voneinander zu erreichen, sind die Anschlusspins vorzugsweise parallel zueinander verlaufend auf einem konzentrisch zum Isolationskörper verlaufenden Kreisbogen gleichmäßig verteilt angeordnet. Alternativ können die Anschlusspins aber auch linear in einer Ebene im Isolationskörper angeordnet sein. Dies kann weitere Fertigungsschritte bei der Schrittmacherherstellung erleichtern. Auch eine lineare Anordnung, bei der zwei oder mehr Reihen von Anschlusspins jeweils zueinander versetzt im Isolationskörper angeordnet sind, kommt in Betracht.

Insbesondere bei der ersten der drei letztgenannten Ausführungsvarianten ist es vorteilhaft, wenn der Kreiskörper eine quer zur Längsrichtung des Anschlusspins oder der Anschlusspins verlaufende Querschnittsfläche aufweist, die rund und vorzugsweise kreisförmig ist.

Der Isolationskörper ist vorzugsweise quer zur Längsrichtung der Pins von einem hülsenartigen, metallischen Flansch eingefasst. Der Flansch besteht vorzugsweise aus einem Material, das bezüglich seiner Zusammensetzung möglichst dem metallischen Gehäuse des Therapiegerätes gleicht. Der Flansch ist entweder aus einem Vollmaterial z. B. durch Drehen oder Fräsen herausgearbeitet oder durch einen geeigneten Sinterprozess hergestellt. Im letztgenannten Fall kann der Flanschkörper von kleinen Poren durchsetzt sein, die jedoch nicht die Hermetizität des Flansches beeinträchtigen. Ein derartiger Flansch kann beispielsweise durch Schweißen mit einem metallischen Gehäuse des Therapiegerätes hermetisch dicht verbunden werden. Flansch und Isolationskörper sind ebenfalls hermetisch dicht miteinander verbunden.

Die Durchführung ist vorzugsweise als Filterdurchführung mit einem Filterkörper ausgebildet. Der Filterkörper besitzt parallel zueinander verlaufende, scheibenförmige Kondensatorelektroden, die abwechselnd mit dem Flansch und mit einem Anschlusspin elektrisch leitend verbunden sind.

Im Zusammenhang mit der letztgenannten Ausführungsvariante erstreckt sich der Flansch vorzugsweise soweit über die innere Stirnfläche des Isolationskörpers hinaus, dass der Flansch auch den Filterkörper wenigstens über den größten Teil seiner Länge umschließt und auf diese Weise leicht elektrisch leitend mit den Kondensatorelektroden des Filterkörpers zu verbinden ist.

Bestehen die Pins aus Niob, Tantal, Titan oder ähnlichen, nicht direkt weich lötbaren Materialien, wird die elektrisch leitfähige Verbindung der Pins mit den Kondensatorelektroden des Filterkörpers über elektrisch leitfähige Kleber oder durch Weichlöten erheblich erleichtert, wenn die Pins mit Goldlot vergoldet sind. Die Vergoldungen können sich auf die Bereiche der Pins beschränken, die für die elektrische Anbindung der Pins mit den Kondensatorelektroden der Filterkörper entscheidend sind.

Ein von der vorliegend beschriebenen Erfindung unabhängiger und selbstständig schutzfähiger Gedanke ist es die Kondensatorelektroden des Filterkörpers mit den Pins und dem Flansch direkt mit Goldlot anzulöten. Dazu bedarf es eines besonders wärmebeständigen Filterkörpers. Eine gefilterte Durchführung kann auf diese Weise kostengünstig in einem einzigen Löt-Schritt gefertigt werden. In diesem Fall kann die Anwendung von weiterem, dichtendem Gold- oder Glaskeramik-Lot entfallen, statt dessen sind die Isolationskörper an geeigneten Stellen beschichtet, z. B. mit Niob, Titan, Tantal oder deren geeignete Legierungen.

Zur Beurteilung der von der Durchführung gebildeten hermetischen Abdichtung des Implantat-Inneren gegenüber der Umgebung ist es vorteilhaft, wenn die Bereiche der Verlötung (mit Glas-, Glaskeramik oder Goldlot) für einen Helium-Lecktest zugänglich sind und nicht durch einen Filterkörper und dessen elektrisch leitfähige Verbindungen zu den Pins und zum Flansch verdeckt werden.

Die Prüfbarkeit der Hermetizität der Durchführung kann auf mehreren Wegen gewährleistet werden:
- Durchgangsöffnung im elektrischen Filterkörper.
- Durchgangsöffnung in einer der elektrisch leitfähigen Verbindungen zwischen dem Filterkörper und den Pins bzw. dem Flansch.
- Der Filterkörper ist in einer Buchse integriert, die über Schweisspunkte mit dem Flansch verbunden ist; der He-Gasdurchtritt ist zwischen den Schweisspunkten gewährleistet.
- Die elektrische Verbindung des Filters mit dem Flansch oder mit den Pins wird durch eine (federnde) Klemmung hergestellt, wobei entweder der Flansch so ausgeformt ist, dass die Federn Bestandteil des Flansches sind oder ein separater Federkörper die elektrische Verbindung zwischen dem Flansch und dem Filter herstellt. Der gewünschte He-Gasdurchtritt erfolgt in diesem Fall zwischen den Klemmpunkten.

In einer Variante sind die Kondensatorelektroden des Filters bereits im Isolationskörper mit integriert, so dass ein separater Filterkörper entfällt. Eine mögliche Ausführungsform ist, dass als Dielektrikum zwischen den Kondensator-elektroden das gleiche keramische Isolationsmaterial (z. B. Al₂O₃) wie an der Oberfläche verwendet wird. In einer weiteren Ausführungsvariante befindet sich ein für die elektrische Filterfunktion angepasstes Material (z. B. BaTiO₃ oder ein anderes keramisches Material hoher Permittivität) zwischen den Kondensatorelektroden, während sich an der Oberfläche ein biokompatibles Isolationsmaterial (z. B. Al₂O₃) befindet.

Um schließlich gleichzeitig eine gute Montierbarkeit und eine gute Dichtigkeit zwischen Flansch und Isolationskörper zu gewährleisten, besitzt der Isolationskörper vorzugsweise einen umlaufenden Absatz in der äußeren Umfangsfläche, der mit einem entsprechenden Absatz in der Innenwand des Flansches zusammenwirkt, wenn die beiden Absätze auf der Umfangsfläche des Isolationskörpers und in der Innenwand des Flansches in Bezug auf die Längsrichtung der Durchführung schräg verlaufen, so dass sich miteinander zusammenwirkende, konische Flächen ergeben, erleichtert der Absatz auch das Zentrieren des Isolationskörpers bezüglich des Flansches.

Die Erfindung soll nun anhand von Ausführungsbeispielen mit Bezug auf die Zeichnungen näher erläutert werden. In den Figuren zeigen:
- Fig. 1a bis 1h:: verschiedene Ausführungsvarianten einer unipolaren Durchführung ohne Filterkörper im Längsschnitt, bzw. einer linearen, multipolaren Durchführung im Querschnitt;
- Fig. 1i bis 1o:: unterschiedliche unipolare Durchführungen als Filterdurchführungen im Längsschnitt, bzw. verschiedene lineare, multipolare Filterdurchführungen im Querschnitt;
- Fig. 1p:: eine gefilterte, unipolare Durchführung mit zwei Varianten des Gaszutritts zur Prüfung der Hermetizität in Draufsicht;
- Fig. 2a bis 2i:: verschiedene Varianten mehrpoliger Durchführungen im Längsschnitt;
- Fig. 2j bis 2o:: verschiedene Varianten mehrpoliger Durchführungen als Filterdurchführungen im Längsschnitt;
- Fig. 3:: eine Ausführung einer unipolaren Filterdurchführung im Längsschnitt mit integrierten Kondensator-Elektroden im Isolationskörper;
- Fig. 4a und 4b:eine: Stirnansicht (Fig. 4a) und eine Seitenansicht (Fig. 4b) einer multipolaren Filterdurchführung gemäß der Erfindung;
- Fig. 4c:: einen Längsschnitt durch die Filterdurchführung gemäß Fig. 4a und 4b; und
- Fig. 5:: einen Herzschrittmacher mit einer erfindungsgemäßen Durchführung.

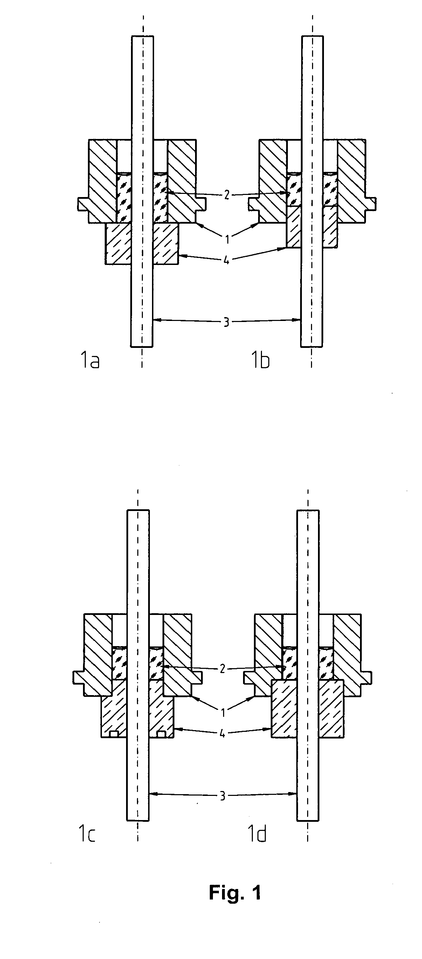

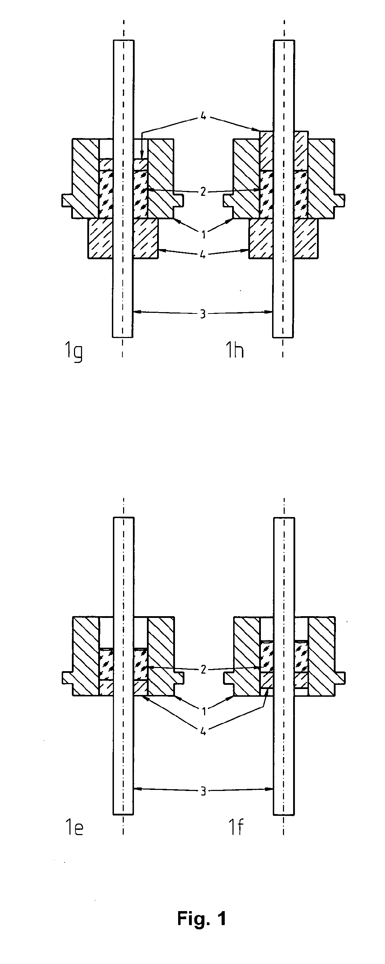

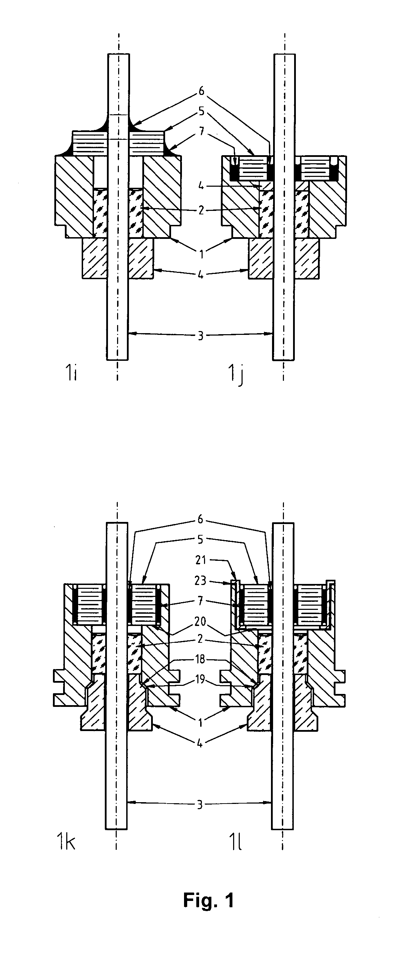

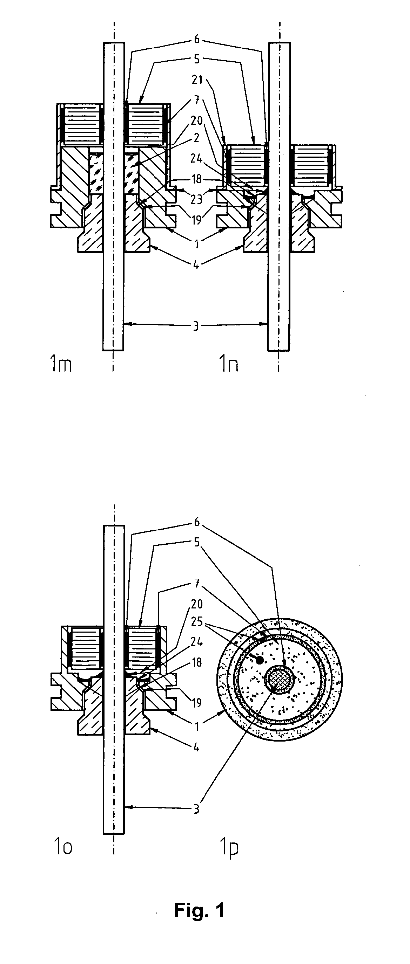

Alle in den Ausführungsbeispielen gemäß Fig. 1a bis 4c dargestellten Durchführungen weisen einen Flansch 1 und wenigstens einen Anschlusspin 3 auf. Der Anschlusspin 3 ist gegenüber dem Flansch 1 mit Hilfe wenigstens eines Isolationskörpers 4 aus Keramik und mit Hilfe von Glas- bzw. Glaskeramik-Lot 2, welches den Pin 3 mit dem Isolationskörper 4 und mit dem Flansch 1 verbindet, gegenüber dem Flansch 1 elektrisch isoliert.

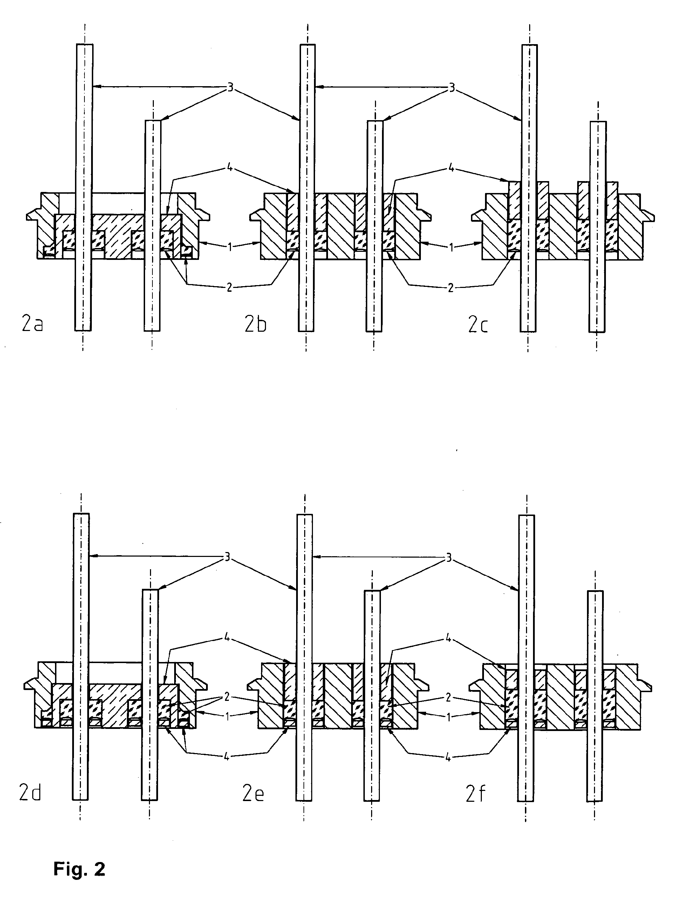

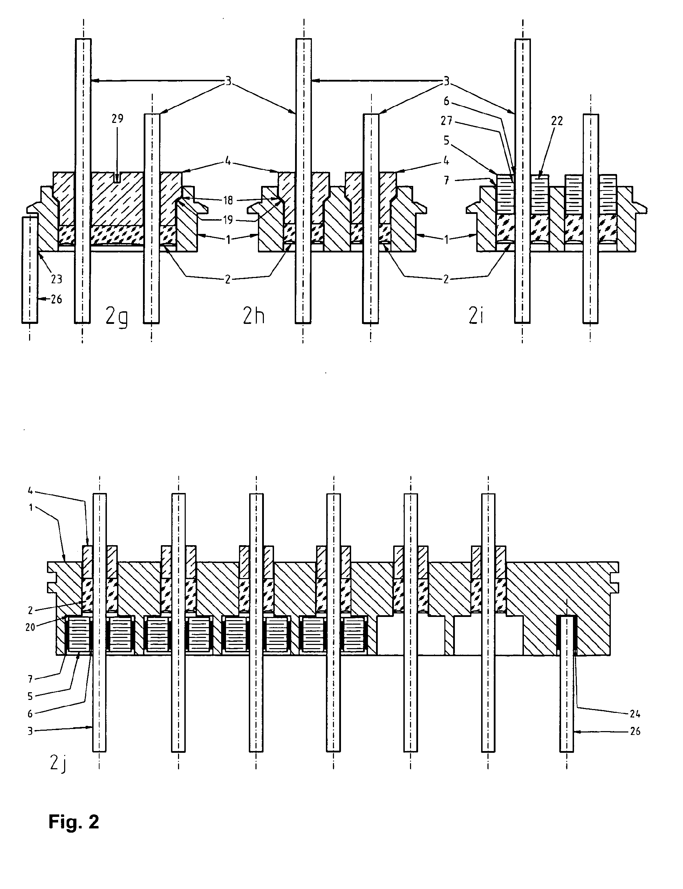

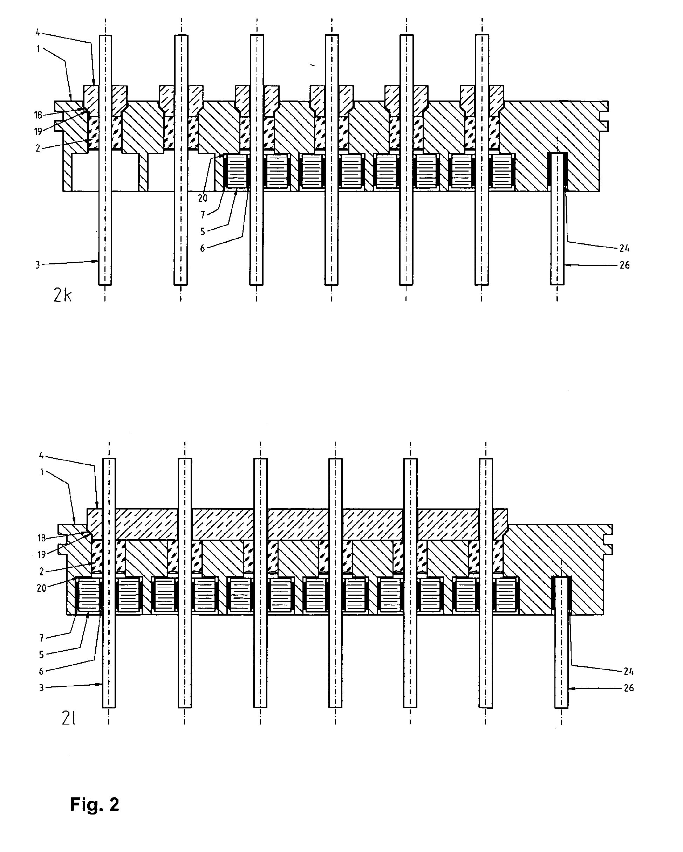

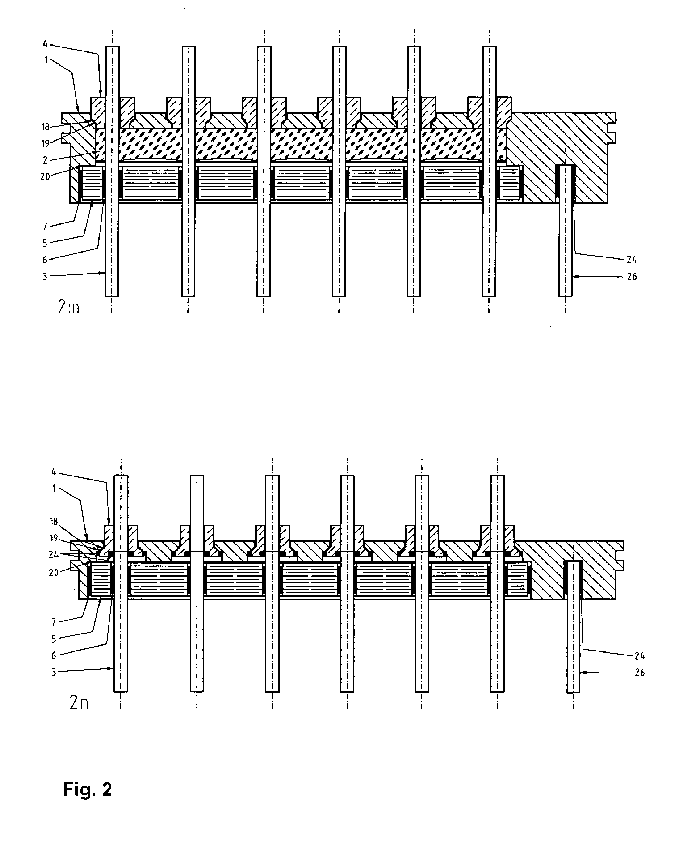

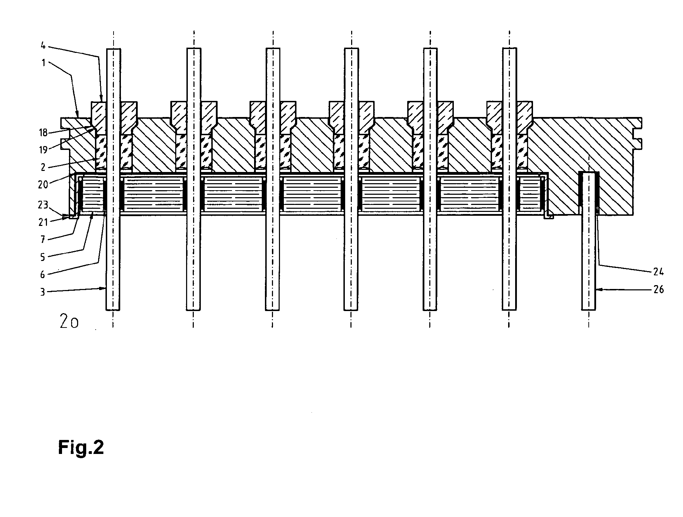

Der Aufbau der jeweils dargestellten Durchführung ergibt sich aus der folgenden Nomenklatur:
Mit Bezugszeichen 1 ist in allen Ausführungsvarianten der Flansch bezeichnet.
Mit Bezugszeichen 2 ist in allen Ausführungsvarianten das Glas- bzw. Glaskeramiklot bezeichnet.
Mit Bezugszeichen 3 sind in allen Ausführungsvarianten die Anschlusspins bezeichnet.
Mit Bezugszeichen 4 sind in allen Ausführungsvarianten Isolationskörper aus Keramik, insbesondere aus Al₂O₃, bezeichnet.
Mit Bezugszeichen 5 ist in den Ausführungsvarianten, die eine Filterdurchführung zeigen, der jeweilige Filterkörper bezeichnet. In diesen Ausführungsvarianten (Fig. 1i-1p und 2j-2o) bezeichnen die Bezugsziffern 6 und 7 ein elektrisch leitfähiges Verbindungsmaterial, wie beispielsweise ein elektrisch leitfähiges Thermoplast oder ein elektrisch leitfähiges (Metall-) Lot.

Fig. 1a zeigt eine hybride Glas/Keramik-Durchführung unipolar bzw. linear multipolar im Querschnitt. Das Glas- bzw. Glaskeramik-Lot 2 verbindet gleichzeitig den Pin bzw. die Pins 3 mit dem Flansch 1 und dem (keramischen) Isolationskörper 4 hermetisch miteinander. Im unipolaren Fall hat der Isolationskörper 4 eine einfache, zylindrische Form. Der Isolationskörper 4 sitzt auf der Stirnfläche des Flansches 1 auf. Als Vorteile ergeben sich, dass das Glas- bzw. Glaskeramik-Lot 2 bei der Verlötung am Herausfliesen nach unten gehindert wird, das die Oberfläche des Isolationskörpers 4 nach außen (in der Figur: auf der Oberseite) biokompatibel ist, keine Beschichtung des Isolationskörpers 4 notwendig ist, eine visuelle Prüfung des Bauteils von innen (in der Figur: von oben nach unten) möglich, der Isolationskörper 4 auf einfache, kostengünstige herzustellen ist und ein guter mechanischer Halt für den Header am herausragenden Isolationskörper 4 gegeben ist.

Die Ausführungsvariante gemäß Fig. 1b ähnelt der aus Fig. 1a, jedoch ragt der Isolationskörper 4 in die Bohrung des Flansches 1 hinein. Hierdurch ergeben sich als zusätzlicher Vorteile eine automatische Zentrierung des Pins 3 relativ zur Bohrung des Flansches und ein kleinerer Isolationskörper 4 mit kleinerem möglichen Pitchmaß (Abstand von Pin zu Pin).

Die Ausführungsvariante gemäß Fig. 1c ähnelt der aus Fig. 1b, jedoch ist der Isolationskörper 4 doppelzylindrisch ausgeführt: ein Zylinder ragt in die Bohrung des Flansches 1 hinein, der andere stößt von außen an die Stirnfläche des Flansches 1 an. Zudem besitzt der Isolationskörper 4 als Variante an der Stirnfläche des größeren Zylinders einen Slot (Vertiefung), der die Isolationsstrecken verlängert und die Hochspannungs-Festigkeit erhöht. Als Vorteile ergeben sich eine zusätzliche Kante zwischen dem Flansch 1 und dem Isolationskörper 4 zur Verminderung der Wechselwirkung zwischen dem Außenbereich und der Glasoberfläche (Stichwort: Dendritenwachstum) sowie eine automatische Zentrierung des Isolationskörpers 4 und des Pins 2 in der Bohrung des Flansches 1. Dafür ist Isolationskörper komplizierter geformt.

Die Ausführungsvariante gemäß Fig. 1d ähnelt der aus Fig. 1a, jedoch ragt der Isolationskörper 4 in einen Absatz im Flansch 1 hinein. Als Vorteile ergeben sich eine automatische Zentrierung des Isolationskörpers 4 und des Pins 2 in der Bohrung des Flansches 1, ein geometrisch einfacher und kostengünstiger Isolationskörper 4, sowie eine zusätzliche Kante zwischen dem Flansch 1 und dem Isolationskörper 4 zur Verminderung der Wechselwirkung zwischen dem Außenbereich und der Glasoberfläche (Stichwort: Dendritenwachstum).

Die Ausführungsvariante gemäß Fig. 1d ähnelt der aus Fig. 1b, jedoch schließt der Isolationskörper 4 bündig mit der Stirnfläche des Flansches 1 ab. Als Vorteil ergibt sich eine kompaktere Bauweise.

Die Ausführungsvariante gemäß Fig. 1f ähnelt der aus Fig. 1e, jedoch liegt die Stirnfläche des Isolationskörpers 4 innerhalb der Bohrung des Flansches 1. Hieraus ergibt sich der Vorteil dass der Header im Sackloch bis zum Isolationskörper 4 mechanisch verzahnt ist.

Die Ausführungsvariante gemäß Fig. 1g ähnelt der aus Fig. 1a, jedoch wird das Glaslot 2 in der Flanschbohrung oben (Innenseite des Implantates) durch einen weiteren Isolationskörper 4 begrenzt. Hieraus ergeben sich als Vorteile eine Eingrenzung des Lotvolumens auf einen definierten Bereich, eine bessere Beherrschung des Lötprozesses (kein Wegfliesen des Glas- bzw. Glaskeramiklotes 2) und damit höhere Ausbeute im Fertigungsprozess, eine Zentrierung des Pins 2 in Bezug zur Bohrung des Flansches 1 an zwei Stellen statt an einer, so dass geforderte Geometrien sicherer eingehalten werden. Allerdings ergibt sich auch ein höherer Bestückungsaufwand durch ein weiteres Bauteil.

Die Ausführungsvariante gemäß Fig. 1h ähnelt der aus Fig. 1g, jedoch ragt der zweite Isolationskörper 4 aus der Bohrung des Flansches 1 heraus. Dies erleichtert die Handhabung des zweiten Isolationskörpers 4 aufgrund seiner Größe.

Die Ausführungsvariante gemäß Fig. 1i ähnelt der aus Fig. 1a, jedoch mit angebrachtem Filterkörper 5 über eine elektrisch leitfähige Stelle 6 am Pin 3 und eine elektrisch leitfähige Stelle 7 am Flansch 1. Die Stoffe 6 und 7 müssen nicht aus dem gleichen Material hergestellt sein. Nicht dargestellt sind optionale Durchgangsöffnungen 25 durch den Filterkörper 5 bzw. die Lötstellen 6 und/oder 7 zur Prüfung der Hermetizität. Auf diese Weise ergibt sich vorteilhaft eine gefilterte Durchführung mit großer Freiheit in der Variabilität der Filtergröße.
Die Ausführungsvariante gemäß Fig. 1j ähnelt der aus Fig. 1i, jedoch befindet sich der Filterkörper 5 in einer Kavität des Flansches 1 und der Pin 3 wird durch zwei Isolationskörper 4 fixiert und das Glas- bzw. Glaskeramik-Lot 2 im Verlauf begrenzt. So ergibt sich eine kompaktere Bauweise

Die Ausführungsvariante gemäß Fig. 1k ähnelt der aus Fig. 1j, jedoch besitzen der Isolationskörper 4 und der Flansch 1 Schrägen 18 und 19, die aufeinander abgestimmt sind und eine besonders gute Zentrierung des Isolationskörpers 4 realtiv zum Flansch 1 bewirken. Außerdem ist der Isolationskörper 4 im weiteren Verlauf außerhalb des Flansches 1 so ausgeformt, dass er für höhere Betriebsspannungen, wie sie z. B. bei Defibrillatoren vorkommen, ausgelegt ist, indem er für längere Strompfade zwischen dem Flansch 1 und dem Pin 3 sorgt. Zudem führt die besondere Ausformung des Isolationskörpers 4 für eine verbesserte Halterung des Headers. Angedeutet ist auch ein Freiraum 20 zwischen dem Filterkörper 7 und dem Flansch 1, der für die Prüfung der Hermetizität einen Gaszutritt zur Glas- bzw. Glaskeramik-Lötstelle ermöglicht.

Die Ausführungsvariante gemäß Fig. 1l ähnelt der aus Fig. 1k, jedoch ist der Filterkörper 5 in einer Buchse 21 eingelötet, die wiederum über Schweißstellen 23 mit dem Flansch 1 verbunden ist. Auf diese Weise ist zur Prüfung der Hermetizität ein Gaszutritt zwischen den Schweißstellen 23 gewährleistet.

Die Ausführungsvariante gemäß Fig. 1m ähnelt der aus Fig. 11, jedoch umfasst die Buchse 21 den Flansch 1.

Die Ausführungsvariante gemäß Fig. 1n ähnelt zwar derjenigen aus Fig. 1m, stellt jedoch keine Ausführungsvariante der beanspruchten Erfindung dar, da statt eines elektrisch isolierenden Glas- bzw. Glaskeramik-Lotes 2 ein metallisches Lot 24 zur Verbindung des Isolationskörpers 4 mit dem Pin 3 und dem

Flansch 1 vorgesehen ist. Hierzu bedarf es einer geeigneten metallischen Beschichtung des Isolationskörpers 4 an mindestens zwei verschiedenen, sich nicht überlappenden Stellen, damit das metallische Lot 24 eine feste haftende, hermetisch dichte Verbindung mit dem Isolationskörper 4 eingehen kann.

Die Ausführungsvariante gemäß Fig. 1o ähnelt der aus Fig. 1n, jedoch ist der Filterkörper 5 direkt in einer Kavität des Flansches 1 mit dem Flansch 1 über die elektrisch leitfähige Verbindung 7 und mit dem Pin 3 über die elektrisch leitfähige Verbindung 6 verbunden.

Fig. 1p zeigt die gleiche Durchführung wie Fig. 1o, jedoch in Draufsicht auf den Filterkörper 5. Angedeutet sind Durchgangsöffnungen 25 zur Prüfung der Hermetizität im Filterkörper 5 bzw. in der elektrisch leitenden Verbindung 7.

Aus den Ausführungsvarianten 1a bis 2o ergibt es sich als gemeinsames Merkmal, dass das jeweilige Glaslot 2 eine Kavität ausfüllt, die von mindestens einem jeweiligen Isolationskörper 4 aus Keramik sowie wenigstens jeweils einem Anschlusspin 3 und ggf. zusätzlich durch einen Flansch 1 definiert ist.

Darüber hinaus sei darauf hingewiesen, dass die in Fig. 1 dargestellten Durchführungen sämtlich unipolare Durchführungen sind. Darüber hinaus können die Querschnitte gemäß Fig. 1a-1o auch als Querschnitte durch lineare, multipolare Durchführungen verstanden werden, die quasi durch Aneinanderreihung einer Reihe von unipolaren Durchführungen zustande kommen.

Die Fig. 1j bis 1p zeigen beispielhaft, dass die dargestellten Durchführungen auch als Filterdurchführungen ausgebildet sein können. Es ist darauf hinzuweisen, dass die Filterdurchführung gemäß Fig. 1i, bis auf den Filterkörper 5 und die elektrisch leitenden Verbindungen 6 und 7, der Durchführung aus Fig. 1a entspricht.

Fig. 2a zeigt eine ungefilterte, hybride Glas/Keramik-Durchführung bi- oder mehrpolar bzw. zweifach linear multipolar im Querschnitt. Das Glas- bzw.

Glaskeramik-Lot 2 verbindet die Pins 3 und den Flansch 1 mit dem (keramischen) Isolationskörper 4 hermetisch miteinander. Der Isolationskörper 4 kann eine einfache, zylindrische Form besitzen, aber auch oval oder langgestreckt sein. Der Isolationskörper 4 befindet sich in einer Kavität des Flansches 1 und sitzt auf einem Absatz im Flansch auf. Alle Pins 3 befinden sich in einem gemeinsamen Isolationskörper 4, mindestens jedoch zwei Pins 3 in je einem Isolationskörper 4. Die Pins 3 können - wie hier angedeutet - unterschiedlich lang ausgeführt sein. In diesem Bild befindet sich die obere Seite der Durchführung im Außenbereich des Implantats. Während der Verlötung bei der Herstellung der Durchführung ist die Orientierung umgekehrt, so dass z. B. das Glas- bzw. Glaskeramiklot 2 auf dem Flansch 1 und dem Isolationskörper 4 aufliegt. Ein Vorteil ist, dass das Glas- bzw. Glaskeramik-Lot 2 bei der Verlötung am Herausfliesen nach oben gehindert wird. Außerdem besitzt der Isolationskörper 4 auf seiner Außenseite (In der Figur: oben) eine biokompatible Oberfläche. Es ist keine Beschichtung des Isolationskörpers 4 notwendig. Außerdem ist eine visuelle Prüfung des Bauteils von innen (in der Figur: Richtung unten) möglich.

Die in Fig. 2b dargestellte Durchführung ist ähnlich der in Fig. 2a dargestellten, jedoch besitzen mindestens zwei Pins 3 jeweils eigene Isolatiönskörper 4 in jeweils separaten Bohrungen des Flansches 1. Die Isolationskörper 4 schließen bündig mit einer Stirnfläche des Flansches 1 ab. Hieraus ergibt sich als Vorteil eine höhere mechanische Stabilität durch die zellenartige Struktur des (metallischen) Flansches 1. Außerdem können die. Isolationskörper 4 mechanisch einfach zylinderförmig und damit universell und kostengünstig ausgeformt sein.

Die in Fig. 2c dargestellte Durchführung ist ähnlich der in Fig. 2b dargestellten, jedoch ragen die Isolationskörper 4 über die Stirnfläche des Flansches 1 hinaus. Als Vorteile ergeben sich eine größere Isolationsstrecken und ein verbesserter mechanischer Halt für den Header des Implantats.

Die in Fig. 2d dargestellte Durchführung ist ähnlich der in Fig. 2a dargestellten, jedoch werden die Glas- bzw. Glaskeramik-Lote 2 durch weitere Isolationskörper 4 auf beiden Seiten begrenzt. Hieraus ergibt sich eine verbesserte Kontrolle des Lotverlaufs und eine Zentrierung der Pins 3 an zwei Stellen.

Die in Fig. 2e dargestellte Durchführung ist ähnlich der in Fig. 2b dargestellten, jedoch werden die Glas- bzw. Glaskeramik-Lote 2 durch weitere Isolationskörper 4 auf beiden Seiten begrenzt. Auch hier ergibt sich eine verbesserte Kontrolle des Lotverlaufs und eine Zentrierung der Pins 3 an zwei Stellen.

Die in Fig. 2f dargestellte Durchführung ist ähnlich der in Fig. 2e dargestellten, jedoch sind mindestens zwei nach außen (in der Zeichnung oben) gerichteten Isolationskörper 4 in den Bohrungen des Flansches 1 versenkt.

Die in Fig. 2g dargestellte Durchführung ist ähnlich der in Fig. 2a dargestellten, jedoch ragt der Isolationskörper 4 aus der Bohrung des Flansches 1 heraus. Zusätzlich weist der Isolationskörper 4 eine Schräge 19 auf, die mit einer Schräge 18 des Flansches 1 korrespondiert und eine besonders gute Zentrierung des Isolationskörpers 4 relativ zum Flansch 1 bewirkt. Der Isolationskörper 4 besitzt zudem einen sog. "Slot" 29, der die Isolationsstrecken zwischen den Pins 3 verlängert und für den Header des Implantates einen weiteren Halt bietet. Ein gemeinsames Glas- bzw. Glaskeramik-Lot 2 verbindet mindestens zwei Pins 3 mit dem Flansch 1 und dem Isolationskörper 4 hermetisch dicht miteinander. Optional ist über eine Verbindung 23 ein Massepin 26 am Flansch 1 angebracht. Die Verbindung 23 wird vorzugsweise durch Schweißen realisiert.

Die in Fig. 2h dargestellte Durchführung ist ähnlich der in Fig. 2c dargestellten, jedoch besitzen der Isolationskörper 4 und der Flansch 1 miteinander korrespondierende Schrägen 19 und 18, die die Zentrierung der Isolationskörper 4 relativ zum Flansch bewirken.

Die in Fig. 2i dargestellte Durchführung ist ähnlich der in Fig. 2c dargestellten, jedoch sind Isolationskörper 4 durch Filterkörper 5 ersetzt. Die Filterkörper 5 besitzen Elektrodenplatten 22 und 27, die abwechselnd mit dem Pin 3 über elektrisch leitende Verbindungen 6 und mit dem Flansch 1 über elektrisch leitende Verbindungen 7 kontaktiert sind. Die elektrisch leitenden Verbindungen 6 und 7 können aus dem gleichen Material bestehen. Für die hermetisch dichte Verbindung des Filterkörpers 5 mit dem Pin 3 und dem Flansch 1 sorgt ein Glas- bzw. Glaskeramik-Lot 2. Vorzugsweise besteht das Dielektrikum des Filterkörpers 5 aus einem biokompatiblen, vorzugsweise keramischen Material oder der Filterkörper 5 ist mit einer biokompatiblen Beschichtung versehen.

Fig. 2j zeigt eine gefilterte, hybride Glas/Keramik-Durchführung, vorzugsweise linear mehrpolar bzw. zwei- oder mehrfach linear multipolar im Querschnitt. Das Glas- bzw. Glaskeramik-Lot 2 verbindet die Pins 3 und den Flansch 1 mit dem vorzugsweise keramischen Isolationskörper 4 hermetisch dicht miteinander. Der Isolationskörper 4 besitzt vorzugsweise eine einfache, zylindrische Form, kann aber auch senkrecht zur dargestellten Querschnittsansicht oval oder langgestreckt sein. Die Isolationskörper 4 befinden sich in Bohrungen des Flansches 1. Alle Pins 3 besitzen jeweils einen eigenen Isolationskörper 4, es können sich aber auch senkrecht zur Querschnittsansicht zwei oder mehr Pins 3 in je einem Isolationskörper 4 befinden. Die Pins 3 können - wie hier nicht angedeutet - unterschiedlich lang ausgeführt sein und/oder an ihren Enden für eine bessere Anbindung geeignet geformt sein, z. B. abgeplattet, nagelförmig, gebogen, usw. In diesem Bild befindet sich die obere Seite der Durchführung im Außenbereich des Implantats. Während der Verlötung bei der Herstellung der Durchführung ist die Orientierung umgekehrt, so dass z. B. das Glas- bzw. Glaskeramiklot 2 auf dem Isolationskörper 4 aufliegt. In diesem Bild werden an einige der Pins 3 elektrische Filterkörper 5 angebracht, bei Bedarf auch an alle Pins 3 oder - in einer ungefilterten Version - an keinem der Pins 3. Die elektrisch leitfähige Verbindung der Filterkörper 5 mit den Pins 3 wird hier über ein metallisches Lot bzw. eine elektrisch leitfähige Masse 6 hergestellt. Ebenso ist die elektrisch leitfähige Verbindung des Filterkörpers 5 mit dem Flansch 1 über das Material 7 ausgeführt, wobei die Materialien 6 und 7 aus dem gleichen Stoff bestehen können. Nicht dargestellt sind Durchgangsöffnungen 25, die durch die Verbindungen 6 oder 7, durch die Filterkörper 5 oder durch die Wandungen des Flansches 1 zu Freiräumen 20 führen, so dass die Hermetizität des fertigen Bauteils geprüft werden kann. Alternativ können die elektrisch leitfähigen Verbindungen 6 und/oder 7 durch Klemmen oder durch Federkraft ausgeführt sein, so dass die beschriebenen Durchgangsöffnungen 25 entfallen können. Optional ist mit dem Flansch 1 ein Massepin 26 über ein elektrisch leitfähiges Material 24, vorzugsweise ein metallisches Lot, verbunden. Auch hier ergibt sich der Vorteil, dass das Glas- bzw. Glaskeramik-Lot 2 bei der Verlötung am Herausfließen nach oben gehindert wird. Weiterer Vorteil ist eine biokompatible Oberfläche des Isolationskörpers 4 auf seiner Außenseite (in der Figur: Richtung oben). Es ist keine Beschichtung des Isolationskörpers 4 notwendig. Außerdem ist eine visuelle Prüfung des Bauteils von innen (in der Figur: Richtung unten) vor dem Anbringen der Filterkörper 5 möglich. Durch den gemeinsamen Flansch 1 ist ein relativ kleines Pitchmaß (Abstand von Pin zu Pin) möglich und zusammen mit separaten Bohrungen mechanisch besonders stabil.

Die Ausführungsvariante gemäß Fig. 2k entspricht weitgehend der aus Fig. 2j, jedoch besitzen die Isolationskörper 4 Schrägen 19, die mit Schrägen 18 des Flansches 1 korrespondieren, so dass die Isolationskörper 4 eine verbesserte Zentrierung in den Bohrungen des Flansches 1 erhalten. Gegenüber der Fig. 2j filtern die Filterkörper 5 Signale an anderen Pins 3.

Die Ausführungsvariante gemäß Fig. 2l entspricht weitgehend der aus Fig. 2k, jedoch werden die Pins 3 durch einen gemeinsamen Isolationskörper 4 geführt. In dieser Ausführungsvariante sind alle Pins 3 mit einem eigenen Filterkörper versehen.

Die Ausführungsvariante gemäß Fig. 2m entspricht weitgehend der aus Fig. 2k, jedoch werden die Isolationskörper 4 und die Pins 3 über ein gemeinsames Glas- bzw. Glaskeramik-Lot 2 hermetisch dicht mit dem Flansch 1 verbunden. In dieser Ausführung wird auch ein gemeinsamer Filterkörper 5 für die Pins 3 verwendet.

Die Ausführungsvariante gemäß Fig. 2n zeigt keine Ausführungsvariante der Erfindung, da gemäß der Ausführungsvariante aus Fig. 2n - die ansonsten derjenigen aus Fig. 2m ähnelt - die Isolationskörper 4 mit den Pins 3 und dem Flansch 1 mit Hilfe eines vorzugsweise metallischen Lotes 24 hermetisch dicht verbunden sind, so dass das Glas- bzw. Glaskeramik-Lot 2 entfallen kann. Hierfür müssen die Isolationskörper 4 eine geeignete Beschichtung aufweisen, so dass sie mit dem Lot 24 benetzt werden können.

Die Ausführungsvariante gemäß Fig. 2o ähnelt derjenigen aus Fig. 2k, jedoch werden die Pins 3 über einen gemeinsamen Filterkörper 5 gefiltert, der mit einer Buchse 21 über ein Material 7 elektrisch leitfähig verbunden ist. Die Buchse 21 ist an geeigneten Stellen 23 elektrisch und mechanisch fest mit dem Flansch 1 verbunden, vorzugsweise durch Schweißverbindungen. Zwischen den Stellen 23 ist ein Gaszutritt in den Freiraum 20 zwischen dem Glas- bzw. Glaskeramik-Lot 2 und dem Filterkörper 5 bzw. der Buchse 21 möglich, so dass zusätzliche Durchgangsöffnungen 25 am Filterkörper 5 oder den Verbindungen 6 und 7 entfallen können und am Bauteil im fertigen Zustand eine Prüfung der hermetischen Dichtigkeit möglich ist.

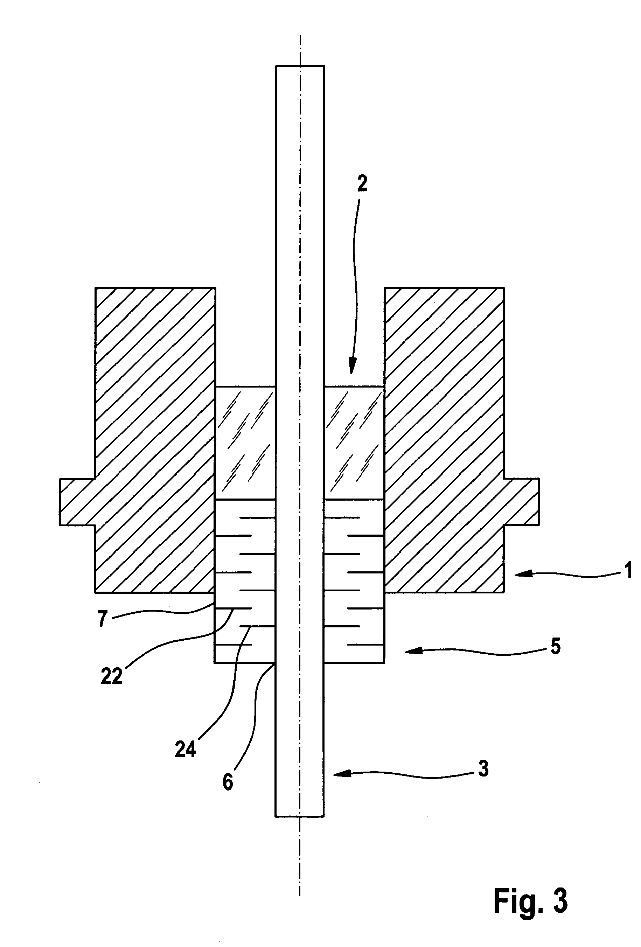

Fig. 3 zeigt schließlich eine Variante einer Filterdurchführung, bei der der Filterkörper 5 gleichzeitig die Funktion des Isolationskörpers übernimmt, also einerseits als Halt für den Anschlusspin 3 dient und andererseits zusammen mit dem Flansch 1 und dem Anschlusspin 3 die Kavität begrenzt, die mit Glaslot 2 gefüllt ist. Fig. 3 zeigt, wie im Sinne der Erfindung auch ein Filterkörper 5 als Isolationskörper wirken kann. In diesem Sinne können die Keramikkörper 4 gemäß der Ausführungsvarianten 1a bis 1f oder 2b, 2c, 2e, 2f, 2h und 2i auch als Filterkörper ausgebildet sein.

Wie Fig. 3 zu entnehmen ist, unterscheidet sich ein Filterkörper 5 von einem reinen Keramikkörper dadurch, dass der Filterkörper 5 elektrisch leitende Kondensatorelektrodenscheiben 22 und 27 aufweist, die abwechselnd jeweils mit dem Anschlusspin 3 und mit dem Flansch 1 elektrisch leitend verbunden sind.

Zwischen den Kondensatorelektrodenscheiben befindet sich ein isolierendes Material, beispielsweise Keramik, das vorzugsweise biokompatibel ist.

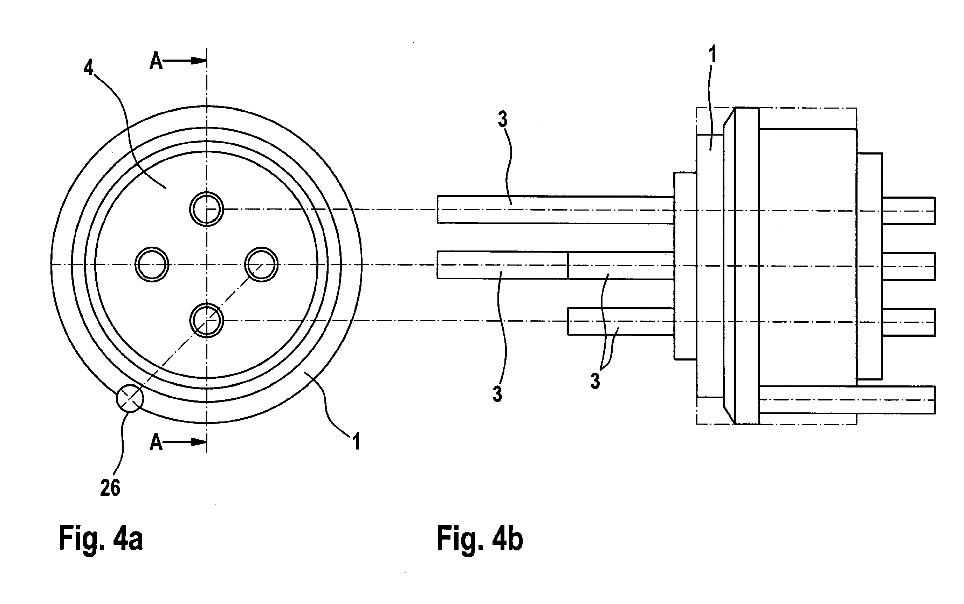

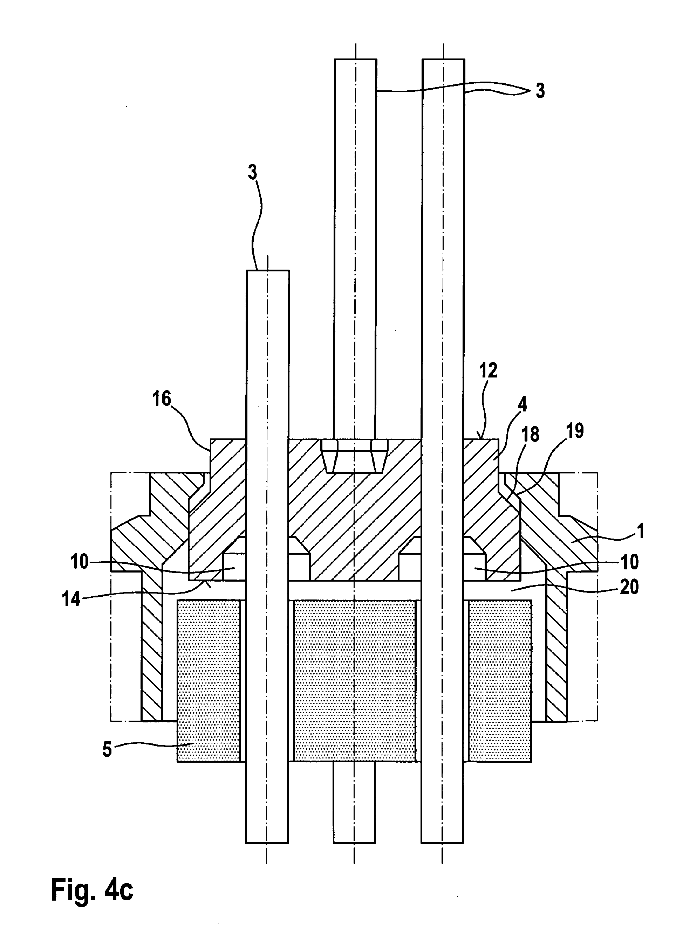

In Fig. 4 ist schließlich eine quadrupolare Filterdurchführung dargestellt. Die Fig. 4a und 4b zeigen die Filterdurchführung in einer Draufsicht bzw. einer Seitenansicht. Fig. 4c ist ein Längsschnitt AA durch die Filterdurchführung (siehe Fig. 4a).

Die Filterdurchführung aus Fig. 4 besitzt vier Anschlusspins 3, die durch entsprechende Durchgangsöffnungen in einem Isolationskörper, der als Keramikkörper 4 ausgebildet ist, hindurchragen.

Der Keramikkörper 4 besteht vorzugsweise aus Al₂O₃. Die Anschlusspins 3 bestehen vorzugsweise aus einer Platin-Iridium-Legierung PtIr 90/10. Die Durchtrittsöffnungen in dem Keramikkörper 4, durch die die Anschlusspins 3 hindurchragen, sind jeweils an einem Längsende derart erweitert, dass zwischen dem jeweiligem Anschlusspin 3 und dem Keramikkörper 4 eine Kavität in Form eines Ringraumes 10 entsteht. Diese Ringräume 10 sind auf einer inneren Stirnfläche 14 des Keramikkörpers 4 angeordnet. Die Ringräume 10 sind bei fertig montierter Durchführung mit Glas- oder Glaskeramiklot gefüllt, welches in Fig. 4 nicht dargestellt ist.

Der Keramikkörper 4 ist von einem Flansch 1 eingefasst, der vorzugsweise aus Titan besteht. In Bezug auf die Gestaltung des Keramikkörpers 4 gemäß der in Fig. 4 dargestellten, beispielhaften Ausführungsvariante ist weiterhin anzumerken, dass der Keramikkörper 4 einen senkrecht zur Längsrichtung der Anschlusspins 3 verlaufenden Querschnitt mit kreisrundem Umfang hat. Die vier Anschlusspins 3 sind parallel zueinander und in Bezug auf den Querschnitt des Keramikkörpers 4 gleichmäßig auf einen Kreisbogen verteilt, der konzentrisch zum übrigen Keramikkörper 4 ist.

Zwei der Anschlusspins 3 sind kürzer als die beiden übrigen Anschlusspins 3, um die Kontaktierung entsprechender Anschlüsse in einem Header eines Implantats zu erleichtern.

In dem in Fig. 4c dargestellten Längsschnitt durch den Keramikkörper 4 ist zu erkennen, dass der Keramikkörper 4 in seiner äußeren Umfangsfläche 16 einen Absatz 18 aufweist, so dass eine konusförmige Mantelfläche entsteht, die mit einem entsprechenden Absatz 19 in dem Flansch 1 korrespondiert.

Der Flansch 1 erstreckt sich in Längsrichtung der Filterdurchführung über die innere Stirnfläche 14 des Keramikkörpers 4 hinaus, so dass der Flansch 1 auf der Innenseite der Filterdurchführung einen Freiraum 20 einschließt, in den ein Filterkörper 5 eingesetzt ist. Der Filterkörper 5 ist optional und kann in dem Fall, in dem eine einfache Durchführung und keine Filterdurchführung benötigt ist, auch weggelassen werden.

Ein typischer Filterkörper 5 weist eine Mehrzahl parallel zueinander und quer zur Längsrichtung der Anschlusspins 3 verlaufender Elektroden auf, von denen jede zweite Elektrode 22 bis in eine äußere Umfangsfläche des Filterkörpers 5 heranragt, während die dazwischen liegenden Elektroden 24 bis an eine jeweilige Durchgangsbohrung für einen jeweiligen Anschlusspin 3 heranreichen; siehe Fig. 3.

Auf der Außenseite des Flansches 1 ist ein Massepin 26 angeordnet, mit dem eine Möglichkeit geschaffen wird, das Implantatsgehäuse mit der Steuerelektronik sicher elektrisch leitend zu kontaktieren.

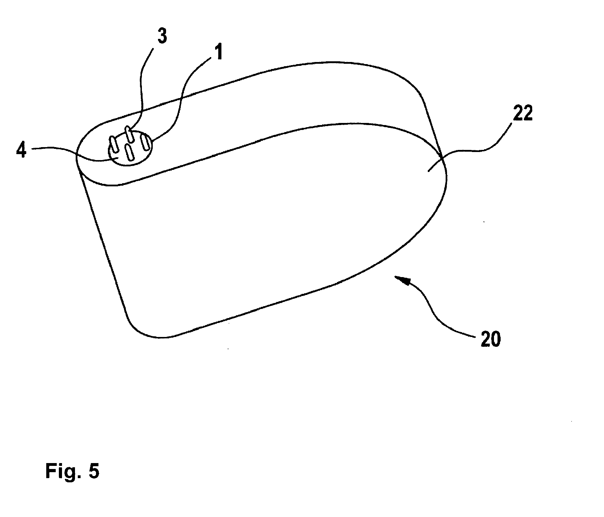

Fig. 5 zeigt schließlich beispielhaft einen Herzschrittmacher 20, dessen metallisches Gehäuse bereits mit einer Filterdurchführung der in Fig. 4a - c abgebildeten Art verschlossen ist. Der Einfachheit halber ist in Fig. 5 nicht der übliche Header eines Herzschrittmachers dargestellt, in dem sich die Anschlussbuchsen für die Elektrodenleitungen befinden. Die elektrischen Kontakte dieser Anschlussbuchsen sind beim fertig gestellten Herzschrittmacher mit den Pins 3 der Filterdurchführung elektrisch leitend verbunden. Die Filterdurchführung - genauer deren Flansch 1 - ist hermetisch dicht mit dem Gehäuse 22 des Herzschrittmachers 20 verbunden, und zwar vorzugsweise durch Verschweißen. Daher ist es vorteilhaft, wenn der Flansch 1 der Filterdurchführung aus dem gleichen Metall besteht, wie das Gehäuse 28 des Herzschrittmachers 20.

Es sei darauf hingewiesen, dass die in den Figuren 1a bis 2o dargestellten Variationen auch in weiteren, hier nicht dargestellten Kombinationen auftreten können.

## Patentansprüche

1. Elektrische Durchführung zum Einsetzen in eine Öffnung eines implantierbaren elektrischen Therapiegerätes mit einem elektrisch isolierenden Isolationskörper (4; 5), durch den wenigstens ein elektrisch leitender Anschlusspin (3) hindurchtritt, der mittels eines Lots (2) hermetisch dicht mit dem Isolationskörper (4; 5) verbunden ist, **dadurch gekennzeichnet, dass** das Lotmaterial Glas- oder Glaskeramik ist.

2. Durchführung nach Anspruch 1, **dadurch gekennzeichnet, dass** der Isolationskörper (4) aus keramischem Material besteht oder ein Al₂O₃ enthaltender Keramikkörper ist.

3. Durchführung nach Anspruch 1 oder 2, **dadurch gekennzeichnet, dass** der Anschlusspin (3) oder die Anschlusspins (3) aus Metall bestehen.

4. Durchführung nach Anspruch 3, **dadurch gekennzeichnet, dass** das Metall der Anschlusspins (3) ein Metall aus der Gruppe Platin, Niob, Tantal und Titan oder eine Legierung dieser Metalle ist oder eine Platin-Iridium-Legierung ist.

5. Durchführung nach einem der Ansprüche 1 bis 4, **dadurch gekennzeichnet, dass** der Isolationskörper (4) Durchgangsöffnungen aufweist, in die jeweils ein Anschlusspin (3) eingesetzt und mit einem von Glas oder Glaskeramik gebildeten Lot (2) mit dem Isolationskörper (4) hermetisch dicht verbunden ist.

6. Durchführung nach Anspruch 5, **dadurch gekennzeichnet, dass** das Glas- oder Glaskeramik-Lot (2) biokompatibel ist.

7. Durchführung nach Anspruch 5, **dadurch gekennzeichnet, dass** Durchgangsöffnungen wenigstens an einem Längsende einen Durchmesser aufweisen, der größer ist, als der Durchmesser eines Anschlusspins (3), so dass zwischen Anschlusspin (3) und erweiterter Durchgangsöffnung ein Ringraum oder eine Kavität (10) besteht, der oder die mit Glas- oder Glaskeramik-Lot (2) gefüllt ist.

8. Durchführung nach Anspruch 7, **dadurch gekennzeichnet, dass** die Durchführung mehrere Anschlusspins (3) und eine entsprechende Anzahl von Durchgangsöffnungen aufweist, die jeweils nur an einem Längsende derart radial erweitert sind, dass sich entsprechende Ringräume (10) bzw. Kavitäten von Anschlusspin (3) zu Anschlusspin (3) auf der gleichen Stirnseite des Isolationskörpers (4) ergeben oder dass sich mindestens eine gemeinsame Kavität zweier oder mehrerer Anschlusspins (3) auf einer Stirnseite des Isolationskörpers (4) ergibt.

9. Durchführung nach Anspruche 8, **dadurch gekennzeichnet, dass** die Durchführung zwei oder mehrere Anschlusspins (3) unterschiedlicher Länge aufweist oder zwei oder mehrere zueinander parallel verlaufende Anschlusspins (3) aufweist.

10. Durchführung nach Anspruch 9, **dadurch gekennzeichnet, dass** die Anschlusspins (3) auf einem konzentrisch zum Isolationskörper (4) verlaufenden Kreisbogen gleichmäßig verteilt sind oder dass die Anschlusspins (3) auf einer Gerade oder mehreren parallel zueinander verlaufenden Geraden gleichmäßig verteilt sind.

11. Durchführung nach einem der Ansprüche 1 bis 10, **dadurch gekennzeichnet, dass** eine senkrecht zu einer Längsrichtung des Anschlusspins (3) verlaufende Querschnittsfläche des Isolationskörper (4) rund und vorzugsweise kreisförmig ist.

12. Durchführung nach einem der Ansprüche 1 bis 11, **dadurch gekennzeichnet, dass** der Isolationskörper (4) in Bezug auf eine Längsrichtung des Anschlusspins (3) in lateraler Richtung von einem hülsenartigen Flansch (1) eingefasst ist.

13. Durchführung nach Anspruch 12, **dadurch gekennzeichnet, dass** der Flansch (1) metallisch leitend ist, wobei der Flansch (1) aus einem Metall besteht, das dem Metall eines Gehäuses eines Therapiegerätes entspricht, für das die Durchführung vorgesehen ist oder der Flansch (1) aus gesintertem Material besteht, welches als Folge des Sinterprozesses zahlreiche Poren enthält.

14. Durchführung nach Anspruch 13, **dadurch gekennzeichnet, dass** die Durchführung als Filterdurchführung ausgebildet ist und einen Filterkörper (5) trägt, der Kondensatorelektrodenscheiben (22, 27) aufweist, die abwechselnd mit dem Flansch (1) und mindestens einem der Anschlusspins (3) elektrisch leitend verbunden sind.

15. Durchführung nach einem der Ansprüche 1 bis 14, **dadurch gekennzeichnet, dass** der Isolationskörper (4) einen umlaufenden Absatz (18) in der äußeren Umfangsfläche aufweist, wobei der umlaufende Absatz (18) schräg ausgebildet ist und einen korrespondierenden Absatz (19) am Flansch (1) als Zentrierhilfe findet.

16. Durchführung nach Anspruch 15, **dadurch gekennzeichnet, dass** der als Zentrierhilfe für den Isolationskörper (4) dienende Absatz (19) am Flansch (1) passend zum schrägen Absatz (18) des Isolationskörpers (4) schräg ausgebildet ist.

17. Implantierbares Elektrotherapiegerät, insbesondere Herzschrittmacher oder Kardioverter/Defibrillator, mit einer Durchführung gemäß einem der Ansprüche 1 bis 16.
